# EUROPÄISCHE PATENTSCHRIFT

(11) **EP 1 239 820 B1**
(45) Veröffentlichungstag und Bekanntmachung des Hinweises auf die Patenterteilung: **16.03.2005**
(21) Anmeldenummer: 00983313.8
(22) Anmeldetag: 13.12.2000
(51) Int. Cl.: A61K 7/135

(54) **BLONDIERMITTEL**
BLEACHING MEANS
PRODUIT DE DECOLORATION

(30) Priorität: 22.12.1999 DE 19961934
(43) Veröffentlichungstag der Anmeldung: 18.09.2002
(73) Patentinhaber: Henkel Kommanditgesellschaft auf Aktien, 40589 Düsseldorf-Holthausen (DE)
(72) Erfinder: HÖFFKES, Horst, 40595 Düsseldorf (DE); NEUHAUS, Winifried, 40625 Düsseldorf (DE)
(86) Internationale Anmeldenummer: PCT/EP2000/012658
(87) Internationale Veröffentlichungsnummer: WO 2001/045658

(56) Entgegenhaltungen:
- WO-A-97/39726
- DE-A- 19 600 216

## Beschreibung

Die Erfindung betrifft ein Blondiermittel für menschliche Haare in Form eines Pulvers oder einer Paste.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhebende Verfahren, die sogenannten Blondierverfahren, im wesentlichen nur hell- bis mittelbraune oder heltiere Haare. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare - insbesondere für die Strähnchenapplikation -werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung üblicherweise mit einer Wasserstoffperoxidlösung vermischt werden, werden im weiteren als "Blondiermittel" bezeichnet. Alle aufgeführten Mengenangaben beziehen sich, soweit nicht anders ausgeführt, ausschließlich auf diese Zubereitungen.

Weder die pastenförmigen noch die pulverförmigen Blondiermittel, die heute auf dem Markt sind, können als optimal angesehen werden. Während die Blondierwirkung auf dem Haar als befriedigend bezeichnet werden kann, besteht doch noch eine Reihe von Nachteilen und Problemen sowohl bei der Herstellung als auch bei der Handhabung dieser Mittel. Bei pastenförmigen Mitteln, die aus Stabilitätsgründen hochviskos eingestellt werden, können insbesondere die Dosierung und das Mischungsverhalten in der Wasserstoffperoxidlösung noch nicht befriedigen. Ferner ist das Staubverhalten bei der Konfektionierung von Pasten ein Problem. Bei pulverförmigen Mitteln stehen das Staubverhalten, sowohl bei der Herstellung als auch bei der Anwendung, sowie ebenfalls das Mischungsverhalten bei der Anwendung im Mittelpunkt der Verbesserungsbemühungen.

In der EP-B1-0 560 088 wurde beispielsweise vorgeschlagen, das Staubverhalten von Blondierpulvern durch Zugabe von Ölen oder flüssigen Wachsen zu verbessern. In der deutschen Anmeldung DE-A1-196 00 216 war weiterhin vorgeschlagen worden, zur Entstaubung spezielle Ether in Mengen von 4 - 20 Gew.-% bezogen auf das gesamte Blondierpulver, einzusetzen. Pastenförmige Blondiermittel wurden in der DE-A1-38 14 356 zur Vermeidung von Staubbildung bei der Verarbeitung beschrieben.

Es wurde nun überraschenderweise gefunden, daß Blondiermittel mit hervorragendem Staubverhalten und weiteren vorteilhaften Eigenschaften erhalten werden, wenn diese Mittel bestimmte Dicarbonsäuren enthalten.

Gegenstand der vorliegenden Erfindung sind daher Mittel zum Blondieren menschlicher Haare auf Basis mindestens einer festen Peroxoverbindung und mindestens eines festen Alkaliträgers, die als Pulver oder als Paste vorliegen, dadurch gekennzeichnet, daß sie eine Dicarbonsäure der Formel (I), 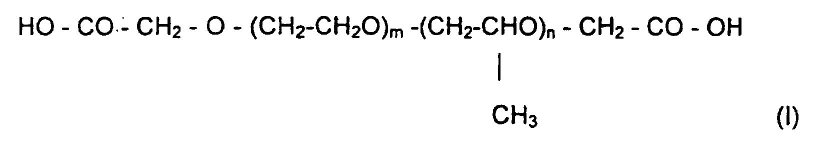 worin m und n jeweils unabhängig voneinander, für 0,1 oder 2 stehen, oder deren Ester enthalten.

Die erfindungsgemäßen Blondiermittel enthalten als erste zwingende Komponente eine feste Peroxoverbindung. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Hamstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Die Peroxoverbindungen sind in den erfindungsgemäßen Blondiermitteln bevorzugt in Mengen von 20-80 Gew.-%, insbesondere in Mengen von 40-70 Gew.-%, enthalten.

Als weitere zwingende Komponente enthalten die erfindungsgemäßen Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxyde, -carbonate,-hydrogencarbonate, -hy-droxycarbonate, -silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Metasilikat und einem Hydroxycarbonat bevorzugt sein.

Die erfindungsgemäßen Blondiermittel enthalten Alkalisierungsmittel bevorzugt in Mengen von 10-30 Gew.-%, insbesondere 15-25 Gew.-%.

Erfindungswesentlich ist die dritte zwingende Komponente der Blondiermittel, eine Dicarbonsäure mit der Formel (I) oder deren Ester, wobei eine Verbindung mit der Formel (I), in der m = 2 und n = 0 ist, 3,6,9-Trioxaundecandisäure, besonders bevorzugt ist. Es ist auch möglich, Gemische aus mehreren Dicarbonsäuren einzusetzen.

Die erfindungsgemäßen Blondiermittel enthalten die Dicarbonsäure der Formel (I) bevorzugt in einer Menge von 1 - 10 Gew.-%.

Weiterhin hat es sich als vorteilhaft erwiesen, wenn die erfindungsgemäßen Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicher-weise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal®B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C₁-C₄-Alkyl-Ethers, "verschlossen". Ein Beispiel für eine solche Verbindung ist das Handelsprodukt Dehypon® LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform® TGI (Henkel)) und Poly(2)glycerinpolyhydroxystearat (Handelsprodukt: Dehymuls®PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beipielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.
Als besonders vorteilhaft haben sich erfindungsgemäße Mittel erwiesen, die nichtionogene grenzflächenaktive Substanzen in Mengen von 0,5 - 10 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.
Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C₈-C₂₂-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C₈-C₂₂-Alkylmono- und oligoglycoside und deren ethoxylierte Analoga. Insbesondere die nichtethoxylierten Verbindungen, die zudem in Pulverform kommerziell erhältlich sind, haben sich als besonders geeignet erwiesen.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrofysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart®F 75 in Abmischung mit Cetearylalkohol erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine,
- Fettalkohole und Fettsäureester,
- Fettsäurealkanolamide,
- Komplexbildner wie EDTA, NTA und Phosphonsäuren,
- Quell- und Penetrationshilfsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

In einer weiteren Ausführungsform können die erfindungsgemäßen Mittel in geringen Mengen als pflegende Komponenten Öle oder Wachse enthalten, wobei die Menge dieser Verbindungen 2 Gew.-% nicht überschreiten sollte, um das Haar nicht übermäßig zu beschweren. Im Sinne der vorliegenden Erfindung umfaßt der Begriff Öle die bekannten fetten und synthetischen Öle, nicht aber Parfümöle, die selbstverständlich in geringen Mengen als Duftstoffe eingesetzt werden können.

Die Herstellung der erfindungsgemäßen Blondiermittel kann nach den üblichen, dem Fachmann bekannten Verfahren erfolgen.

Ein bevorzugtes Verfahren besteht darin, die anorganischen, als Feststoff vorliegenden Komponenten, gegebenenfalls nach Mischung z.B. in einem Drais-Mischer, vorzulegen und mit dem grenzflächenaktiven Mittel zu besprühen. Dies erfolgt bevorzugt bei Raumtemperatur, d. h. bei Temperaturen unterhalb von ca. 30 °C; lediglich wenn die gewählten staubbindenden Komponenten bei diesen Temperaturen nicht als Flüssigkeit vorliegen, wird man erhöhte Temperaturen anwenden.

Ein weiteres Herstellungsverfahren für die erfindungsgemäßen Blondiermittel ist das Vermahlen aller Komponenten in einer Kugelmühle, einer Ringwalzenmühle oder insbesondere einer Spindelmühle.

Schließlich ist es möglich, die pulverförmigen Blondiermittel durch Mischen aller Komponenten und die anschließende Behandlung, bevorzugt bei erhöhten Temperaturen, im Wirbelbett herzustellen.

Für die Anwendung der erfindungsgemäßen Mittel auf dem Haar werden die Blondiermittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12prozentige Lösungen in Wasser verwendet. Die Mengenverhältnisse von Blondiermittel und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

### Beispiele

Es wurden 6 Blondiermittel zubereitet, deren Zusammensetzung in Gew.-% in Tabelle 1 angegeben sind. In den Beispielen A1 bis A5 wurden verschiedene Blondiermittel mit unterschiedlichen Gehalten an 3,6,9-Trioxaundecansäure entstaubt. Das Beispiel V ist ein Vergleichszubereitung ohne Dicarbonsäure.

Alle Mittel außer der Vergleichszubereitung V waren staubfrei.

Alle Mittel ergaben beim Vermischen mit einem 6%igen Entwickler (Marktprodukt Poly Brillance) im Verhältnis 1:1 innerhalb kurzer Zeit homogene Mischungen (Vergleichzubereitung V wurde im Verhältnis 0,92:1 gemischt).

Dunkelblonde Haarsträhnen (Fischbach + Miller, Code 6923) von ca. 0,5g Gewicht wurden mit jeweils ca. 2g Blondiermittel 30min lang blondiert. Im Anschluß erfolgte eine visuelle Beurteilung im Vergleich zu Rezepturen, die keine Entstaubungsmittel enthielten. Eine Beeinträchtigung der Aufhellwirkung der erfindungsgemäßen Mittel konnte nicht festgestellt werden.

**Tabelle 1:**

| Komponenten | A1 | A2 | A3 | A4 | A5 | V |
|---|---|---|---|---|---|---|
| Ammoniumperoxidisulfat | 56,5 | - | - | - | - | - |
| Kaliumperoxidisulfat | - | 56,5 | 54,5 | - | - | 57,4 |
| Natriumperoxidisulfat | - | - | - | 56,5 | 46,5 | - |
| Natriummetasilikat | 18,0 | 18,0 | 18,0 | 18,0 | 18,0 | 19,4 |
| Magnesiumhydroxycarbonat | 3,0 | 3,0 | 3,0 | 3,0 | 3,0 | 3,2 |
| Natriumchlorid | 5,0 | 4,0 | 5,0 | 1,0 | - | 2,2 |
| Kaliumsulfat | - | - | - | - | 10,0 | - |
| Natriumstearat | 11,0 | 11,0 | 11,0 | 11,0 | 11,0 | 11,8 |
| Pyrogenes Siliziumdioxid | 2,5 | 2,5 | 2,5 | 2,5 | 2,5 | 2,7 |
| Dinatriumdihydrogenethylendiamintetraacetat | 1,0 | 1,0 | 1,0 | 1,0 | 1,0 | 1,1 |
| 3,6,9-Trioxaundecandisäure | 1,0 | 2,0 | 5,0 | 7,0 | 8,0 | - |
| Trinatriumphosphat | 2,0 | 2,0 | - | - | - | 2,2 |
| Gesamtmenge | 100 | 100 | 100 | 100 | 100 | 100 |

## Patentansprüche

1. Mittel zum Blondieren menschlicher Haare auf Basis mindestens einer festen Peroxoverbindung und mindestens eines festen Alkaliträgers, das als Pulver oder Paste vorliegt, **dadurch gekennzeichnet, daß** es mindestens eine Dicarbonsäure der Formel (I) 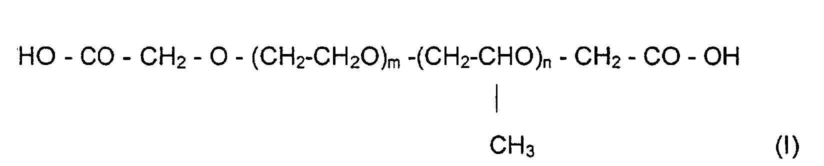 worin m und n jeweils unabhängig voneinander für 0,1 oder 2 stehen, oder deren Ester enthält.

2. Mittel nach Anspruch 1, **dadurch gekennzeichnet, daß** es eine anorganische feste Peroxoverbindung enthält.

3. Mittel nach einem der Ansprüche 1 bis 2, **dadurch gekennzeichnet, daß** die Verbindung der Formel (I) 3,6,9-Trioxaundecandisäure ist.

4. Mittel nach einem der Ansprüche 1 bis 3, **dadurch gekennzeichnet, daß** die Verbindung der Formel (I) in einer Menge von 0,1 - 10 Gew.-%, insbesondere in einer Menge von 1 bis 8 Gew.-%, enthalten ist.

5. Mittel nach einem der Ansprüche 1 bis 4, **dadurch gekennzeichnet, daß** es eine nichtionogene grenzflächenaktive Substanz in Mengen von 0,5 - 10 Gew.-% enthält.

6. Mittel nach einem der Ansprüche 1 bis 5, **dadurch gekennzeichnet, daß** es eine nichtionogene grenzflächenaktive Substanz, ausgewählt aus
- alkoxylierten Fettalkoholen und Fettsäuren mit 8 bis 22 Kohlenstoffatomen und 1 bis 30 Ethylenoxid- und/oder Propylenoxid-Einheiten, die eine Alkylendgruppe, insbesondere eine Methylgruppe, am Ende der Alkoxygruppenkette aufweisen können,
- alkoxylierten Mono-, Di- und Triglyceriden,
- Polyglycerinestern und alkoxylierten Polyglycerinestern,
- Sorbitan-Fettsäureestern und alkoxylierten Sorbitan-Festtsäureestem,
- Alkylphenolalkoxylaten mit 6 bis 21 Kohlenstoffatomen in der Alkylkette und 0 bis 30 Ethylenoxid- und/oder Propylenoxid-Einheiten
enthält.

7. Mittel nach einem der Ansprüche 1 bis 6, **dadurch gekennzeichnet, daß** es bei Raumtemperatur feste Seifen, insbesondere Natriumstearat, in Mengen von 5-20 Gew.-%, insbesondere 10-15 Gew.-%, bezogen auf das gesamte Blondiermittel, enthält.

8. Mittel nach einem der Ansprüche 1 bis 7, **dadurch gekennzeichnet, daß** es bis zu 2 Gew.-% einer öligen oder wachsartigen Komponente enthält.

9. Verwendung eines der Mittel der Ansprüche 1 bis 8 zum Blondieren menschlicher Haare.

## Claims

1. A powder-form or paste-form preparation for blonding human hair based on at least one solid peroxo compound and at least one solid alkalizing agent, **characterized in that** it contains at least one dicarboxylic acid corresponding to formula (I): 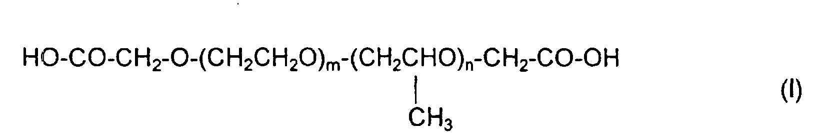 where m and n independently of one another stand for 0, 1 or 2, or an ester thereof.

2. A preparation as claimed in claim 1, **characterized in that** it contains an inorganic solid peroxo compound.

3. A preparation as claimed in claim 1 or 2, **characterized in that** the compound of formula (I) is 3,6,9-trioxaundecanedioic acid.

4. A preparation as claimed in any of claims 1 to 3, **characterized in that** the compound of formula (I) is present in a quantity of 0.1 to 10% by weight and more particularly in a quantity of 1 to 8% by weight.

5. A preparation as claimed in any of claims 1 to 4, **characterized in that** it contains a nonionic surfactant in quantities of 0.5 to 10% by weight.

6. A preparation as claimed in any of claims 1 to 5, **characterized in that** it contains a nonionic surfactant selected from
- alkoxylated fatty alcohols and fatty acids containing 8 to 22 carbon atoms and 1 to 30 ethylene oxide and/or propylene oxide units which may have a terminal alkyl group, more particularly a methyl group, at the end of the alkoxy group chain,
- alkoxylated mono-, di- and triglycerides,
- polyglycerol esters and alkoxylated polyglycerol esters,
- sorbitan fatty acid esters and alkoxylated sorbitan fatty acid esters,
- alkylphenol alkoxylates with 6 to 21 carbon atoms in the alkyl chain and 0 to 30 ethylene oxide and/or propylene oxide units.

7. A preparation as claimed in any of claims 1 to 6, **characterized in that** it contains soaps solid at room temperature, more particularly sodium stearate, in quantities of 5 to 20% by weight and more particularly in quantities of 10 to 15% by weight, based on the blonding preparation as a whole.

8. A preparation as claimed in any of claims 1 to 7, **characterized in that** it contains up to 2% by weight of an oily or wax-like component.

9. The use of the preparation claimed in claims 1 to 8 for blonding human hair.

## Revendications

1. Agent de décoloration de cheveux humains, à base d'au moins un composé peroxo solide et d'au moins un support alcalin solide, se présentant sous forme de poudre ou de pâte, **caractérisé en ce qu'**il contient au moins un acide dicarboxylique de formule (I) 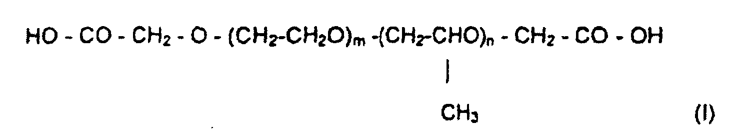 dans laquelle m et n valent indépendamment l'un de l'autre 0, 1 ou 2, ou un de ses esters.

2. Agent selon la revendication 1, **caractérisé en ce qu'**il contient un composé peroxo solide inorganique.

3. Agent selon l'une quelconque des revendications 1 à 2, **caractérisé en ce que** le composé de formule (I) est l'acide 3,6,9-trioxaundécanedioïque.

4. Agent selon l'une quelconque des revendications 1 à 3, **caractérisé en ce qu'**il contient le composé de formule (I) dans une proportion de 0,1-10 % en poids, en particulier dans une proportion de 1 à 8 % en poids.

5. Agent selon l'une quelconque des revendications 1 à 4, **caractérisé en ce qu'**il contient une substance tensioactive non ionogène dans une proportion de 0,5-10 % en poids.

6. Agent selon l'une quelconque des revendications 1 à 5, **caractérisé en ce qu'**il contient une substance tensioactive non ionogène choisi parmi
- les alcools gras et acides gras alcoxylés comprenant 8 à 22 atomes de carbone et 1 à 30 unités oxyde d'éthylène et/ou oxyde de propylène qui peuvent présenter, à l'extrémité de la chaîne alcoxy, un groupe terminal alkyle, en particulier un groupe méthyle,
- les mono-, di- et triglycérides alcoxylés,
- les esters de polyglycérol et les esters de polyglycérol alcoxylés,
- les esters d'acide gras et de sorbitane et les esters d'acide gras et de sorbitane alcoxylés,
- les alcoxylates d'alkylphénols dont la chaîne alkyle comprend 6 à 21 atomes de carbone et 0 à 30 unités oxyde d'éthylène et/ou oxyde de propylène.

7. Agent selon l'une quelconque des revendications 1 à 6, **caractérisé en ce qu'**il contient des savons qui sont solides à la température ambiante, en particulier le stéarate de sodium, dans une proportion de 5-20 % en poids, en particulier de 10-15 % en poids, par rapport à la totalité de l'agent décolorant.

8. Agent selon l'une quelconque des revendications 1 à 7, **caractérisé en ce qu'**il contient jusqu'à 2 % en poids d'un composant de type huile ou cire.

9. Utilisation d'un agent selon l'une quelconque des revendications 1 à 8, pour la décoloration de cheveux humains.
